# EUROPEAN PATENT APPLICATION

(11) **EP 4 768 482 A1**
(43) Date of publication of application: **01.07.2026**
(21) Application number: 24872899.0
(22) Date of filing: 25.09.2024
(51) Int. Cl.: C07D 417/12, A61K 31/427, A61K 31/496, A61P 35/00, C07D 235/32, C07D 401/12, C07D 409/14, C07D 401/14

(54) **THIOBENZIMIDAZOLE DERIVATIVE OR PHARMACEUTICALLY ACCEPTABLE SALT THEREOF, AND USE THEREOF**

(30) Priority: 26.09.2023 KR 20230129755; 24.09.2024 KR 20240129304
(71) Applicant: Korea University Research and Business Foundation, Seoul 02841 (KR)
(72) Inventor: SEO, Jae Hong, Gwacheon-si Gyeonggi-do 13803 (KR); NAM, Kee Dal, Seoul 02467 (KR); KIM, Ji Young, Seoul 08226 (KR); KIM, Yoon Jae, Paju-si Gyeonggi-do 10887 (KR); JUNG, Eun-Sun, Incheon 21122 (KR); KANG, Yong Koo, Seoul 04426 (KR)
(74) Representative: Cousens, Nico
(86) International application number: PCT/KR2024/014476
(87) International publication number: WO 2025/071191

(57) **Abstract**

The present invention relates to a thiobenzimidazole derivative or a pharmaceutically acceptable salt thereof, and a composition for preventing or treating cancer, comprising the derivative as an active ingredient. The thiobenzimidazole derivative of the present invention exhibits cell toxicity by blocking the cell cycle of a cancer cell and inducing apoptosis when administered to an individual, as the derivative inhibits tubulin polymerization by being activated in the cancer cell, and, thus, the derivative can be used for prevention or treatment of cancer, desirably for prevention or treatment of triple-negative breast cancer.

## Description

### TECHNICAL FIELD

The disclosure relates to a thiobenzimidazole derivative or a pharmaceutically acceptable salt thereof, and a use thereof.

### BACKGROUND ART

Patients with triple-negative breast cancer (TNBC; ER-, PR-, HER2-) account for 10 to 15% of all breast cancer patients, and since hormone receptors (estrogen receptor (ER), progesterone receptor (PR)) and HER2 protein are absent, these patients do not benefit from hormone therapy or HER2-targeted therapeutics. Currently, the standard treatment for triple-negative breast cancer is entirely dependent on general cytotoxic anticancer agents (Taxane-based or Anthracycline-based). Because there is no established targeted therapeutic agent, treatment strategies for other subtypes are not as diverse compared to breast cancer. More seriously, after surgery or anticancer treatment, recurrence occurs within 2 to 3 years in most patients, and metastasis to other organs, such as the lungs, the liver, the brain, and the bones, is easily induced, which affects a decrease in patient survival rates.

The 5-year overall survival of patients diagnosed with stage III (stage-III) is 55% or less, and for patients with advanced-stage cancer in which cancer metastasis has already progressed, the 5-year overall survival is very low at 30% or less. Most of these patients ultimately die within several years, making it a very serious disease.

A microtubule is a major component of a cytoskeleton, and is composed of a tubulin heteropolymer consisting of an α subunit and a β subunit. Microtubules perform various cellular functions such as intracellular transport, polarity maintenance, intracellular signal transduction, cell migration, and proliferation. During mitosis of a cell, a spindle fiber is formed to perform a process in which chromosomes are aligned at the center of the cell and then separated toward both poles. When the spindle fiber fails to function properly, cell division is inhibited and apoptosis occurs, and thus, the spindle fiber has attracted attention as a target for anticancer agents.

Drugs targeting microtubules are largely divided into two groups: drugs that stabilize microtubules and drugs that destabilize microtubules. First, microtubule stabilizers include Taxane, Paclitaxel (Taxol), and Docetaxel, and these stabilizers function to prevent depolymerization of microtubules and to enhance polymerization. Most microtubule-stabilizing substances bind to a Taxane-binding site or an overlapping site of β-tubulin. Second, microtubule destabilizers include colchicine and vinca alkaloid, and these destabilizers bind to a colchicine binding site or a vinca binding site. Drugs targeting the microtubules themselves exhibit effects at lower concentrations than drugs affecting microtubule polymers, and the point that these drugs ultimately inhibit cell mitosis is the same. Therefore, there is a need to develop potential tubulin polymerization inhibitors as anticancer agents.

Meanwhile, flubendazole and albendazole are drugs marketed as anthelmintics as tubulin polymerization inhibitors, but apoptosis by cell cycle arrest is confirmable, and since an apoptotic effect has been confirmed even on slowly growing cancer cells, flubendazole and albendazole are attracting attention as therapeutic agents for various cancers. However, flubendazole and albendazole have a limitation in that absorption in the body is difficult due to low solubility in water, and side effects such as an increase in liver enzyme levels appear upon ingestion in large amounts, and thus development of derivatives having a novel structure is required.

Accordingly, the present inventors confirmed that a novel thiobenzimidazole derivative, or a pharmaceutically acceptable salt thereof, as a tubulin polymerization inhibitor, induces apoptosis by causing cell cycle arrest in cancer cells, and completed the disclosure.

A related prior art includes Indian Journal of Chemistry, Vol. 52B, April 2013, pp 535-545.

### DISCLOSURE OF THE INVENTION

### TECHNICAL GOALS

A technical problem to be achieved by the disclosure is to provide a thiobenzimidazole derivative or a pharmaceutically acceptable salt thereof.

A technical problem to be achieved by the disclosure is to provide a method of manufacturing the thiobenzimidazole derivative, or the pharmaceutically acceptable salt thereof.

In addition, another object of the disclosure is to provide a pharmaceutical composition for preventing or treating cancer, including, as an active ingredient, the thiobenzimidazole derivative or the pharmaceutically acceptable salt thereof.

However, technical goals to be achieved are not limited to those described above, and other goals not mentioned above are clearly understood by one of ordinary skill in the art from the following description.

### TECHNICAL SOLUTIONS

In order to solve the above problem, the disclosure provides a thiobenzimidazole derivative represented by [Formula 1], a racemate thereof, an isomer thereof, a solvate thereof, or a pharmaceutically acceptable salt thereof: 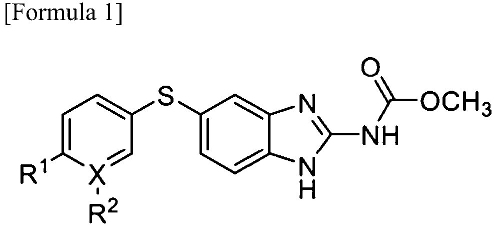 in Formula 1,
X is any one selected from C and N,
R¹ is any one selected from a substituted or unsubstituted C₃ to C₂₀ cycloalkyl, a substituted or unsubstituted C₃ to C₂₀ heterocycloalkyl, a substituted or unsubstituted C₃ to C₂₀ aryl, and a substituted or unsubstituted C₃ to C₂₀ heteroaryl,
when the cycloalkyl, the heterocycloalkyl, the aryl, or the heteroaryl is substituted, the cycloalkyl, the heterocycloalkyl, the aryl, or the heteroaryl is substituted with one or more selected from a heterocycloalkyl group, a C₁ to C₆ alkyl group, a hydroxy group, an alkylacyl group, -CF₃, and a dimethylamino group, and
R² is a hydrogen or a halogen.

As an embodiment of the disclosure, in [Formula 1],

R¹ may be any one selected from the group consisting of a substituted or unsubstituted piperidine, a substituted or unsubstituted morpholine, a substituted or unsubstituted piperazine, and a substituted or unsubstituted thiophene, and

when the piperidine, the morpholine, the piperazine, or the thiophene is substituted, the piperidine, the morpholine, the piperazine, or the thiophene may be substituted with one or more selected from a morpholine group, a methyl group, a hydroxy group, an isobutylacyl group, -CF₃, and a dimethylamino group.

As another embodiment of the disclosure, the thiobenzimidazole derivative represented by [Formula 1] may be any one selected from the group consisting of the following compounds:

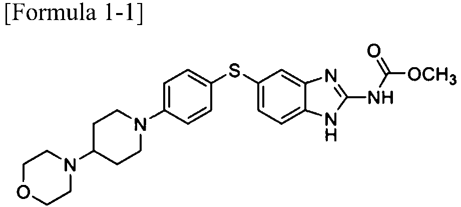

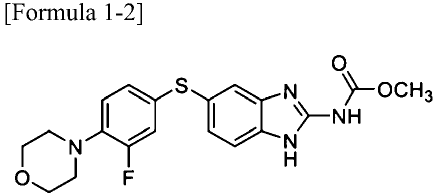

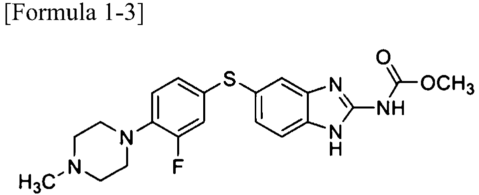

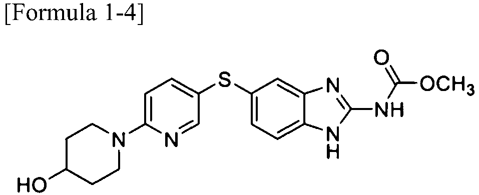

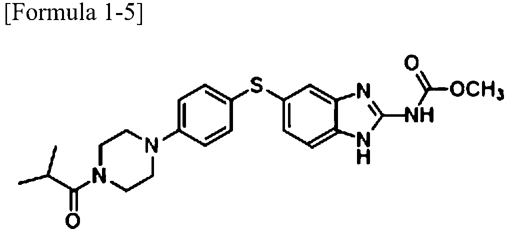

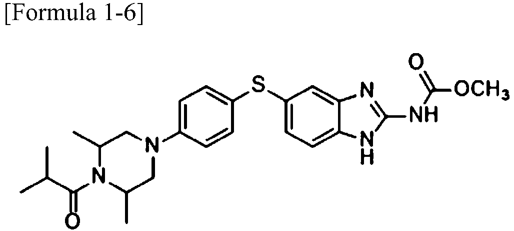

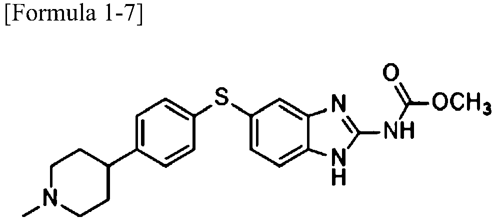

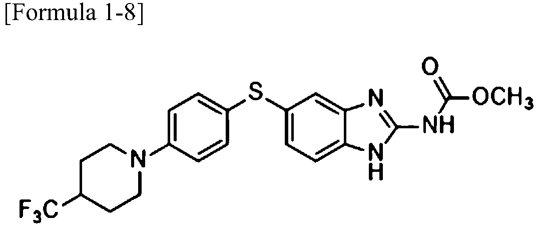

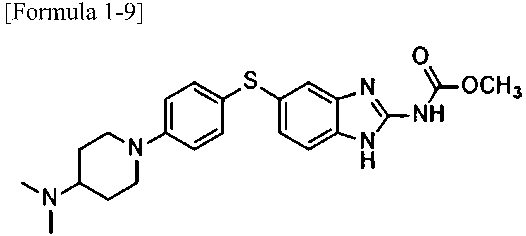

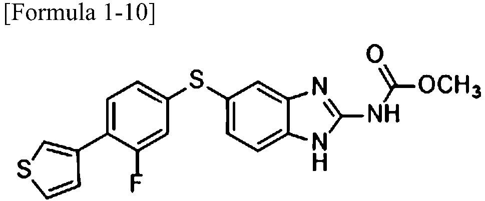

As yet another embodiment of the disclosure, the thiobenzimidazole derivative may inhibit tubulin polymerization.

As yet another embodiment of the disclosure, the pharmaceutically acceptable salt of the thiobenzimidazole derivative may be one or more selected from the group consisting of a hydrochloride, a bromate, a sulfate, a phosphate, a nitrate, a citrate, an acetate, a lactate, a tartrate, a maleate, a gluconate, a succinate, a formate, a trifluoroacetate, an oxalate, a fumarate, a glutarate, an adipate, a methanesulfonate, a benzenesulfonate, a p-toluenesulfonate, a camphorsulfonate, a sodium salt, a potassium salt, a lithium salt, a calcium salt, and a magnesium salt, and preferably may be a hydrochloride.

In addition, the disclosure provides a composition for preventing or treating cancer, the composition including, as an active ingredient, the thiobenzimidazole derivative or a pharmaceutically acceptable salt thereof.

In addition, the disclosure provides a method of preventing or treating cancer, the method including administering the thiobenzimidazole derivative or a pharmaceutically acceptable salt thereof to a subject.

In addition, the disclosure provides a use of the thiobenzimidazole derivative or a pharmaceutically acceptable salt thereof for the manufacture of a medicament for preventing or treating cancer.

In addition, the disclosure provides a method of diagnosing cancer, the method including administering the thiobenzimidazole derivative or a pharmaceutically acceptable salt thereof to a subject.

In addition, the disclosure provides a use of the thiobenzimidazole derivative or a pharmaceutically acceptable salt thereof for the manufacture of a medicament for diagnosing cancer.

As an embodiment of the disclosure, the pharmaceutical composition may induce apoptosis by causing cell cycle arrest in cancer cells.

As another embodiment of the disclosure, the cancer may be one or more selected from the group consisting of skin cancer, breast cancer, uterine cancer, esophageal cancer, gastric cancer, brain tumor, colon cancer, rectal cancer, colorectal cancer, lung cancer, ovarian cancer, cervical cancer, endometrial cancer, vulvar cancer, renal cancer, hematological cancer, pancreatic cancer, prostate cancer, testicular cancer, laryngeal cancer, head and neck cancer, thyroid cancer, hepatic cancer, bladder cancer, osteosarcoma, lymphoma, leukemia, thymic cancer, urethral cancer, and bronchial cancer, and preferably may be breast cancer, and more preferably may be HER-2 positive breast cancer or triple-negative breast cancer.

### EFFECTS OF THE INVENTION

The disclosure relates to a thiobenzimidazole derivative, or a pharmaceutically acceptable salt thereof, and a composition for preventing or treating cancer including the derivative as an active ingredient, wherein the thiobenzimidazole derivative of the disclosure is activated in cancer cells to inhibit tubulin polymerization, and exhibits cytotoxicity by blocking cancer cell cycle and inducing apoptosis upon administration to a subject, and thus the thiobenzimidazole derivative may be used for prevention or treatment of cancer, preferably for prevention or treatment of HER-2 positive breast cancer or triple-negative breast cancer.

Effects of the disclosure are not limited to those mentioned above, and other effects not mentioned will be clearly understood by those skilled in the art from the description below.

### BRIEF DESCRIPTION OF DRAWINGS

FIG. 1 illustrates experimental results of treating the human breast cancer cell lines MDA-MB-231 and JIMT-1 with thiobenzimidazole derivatives of Formula 1-1 to Formula 1-10.
FIG. 2 illustrates treating the MDA-MB-231 and JIMT-1 cell lines with thiobenzimidazole derivatives (Formula 1-1 to 1-4) of the disclosure and deriving IC50 values.

### BEST MODE FOR CARRYING OUT THE INVENTION

The present inventors conducted extensive research on a thiobenzimidazole derivative, or a pharmaceutically acceptable salt thereof, and as a result, confirmed anticancer activity of the derivative and completed the disclosure.

More specifically, it was confirmed that the thiobenzimidazole derivative, or the pharmaceutically acceptable salt thereof, is activated in cancer cells to inhibit tubulin polymerization, thereby blocking cancer cell cycle and inducing apoptosis, thus exhibiting cytotoxicity.

From the above results, the disclosure provides a thiobenzimidazole derivative represented by [Formula 1], or a pharmaceutically acceptable salt thereof.

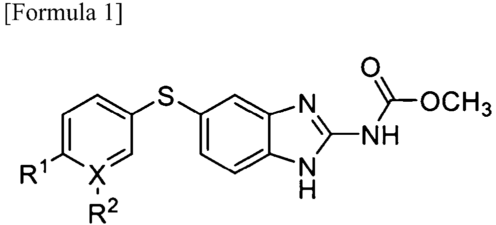

In Formula 1,
X is any one selected from C and N,
R¹ is any one selected from a substituted or unsubstituted C₃ to C₂₀ cycloalkyl, a substituted or unsubstituted C₃ to C₂₀ heterocycloalkyl, a substituted or unsubstituted C₃ to C₂₀ aryl, and a substituted or unsubstituted C₃ to C₂₀ heteroaryl,
when the cycloalkyl, the heterocycloalkyl, the aryl, or the heteroaryl is substituted, the cycloalkyl, the heterocycloalkyl, the aryl, or the heteroaryl is substituted with one or more selected from a heterocycloalkyl group, a C₁ to C₆ alkyl group, a hydroxy group, an alkylacyl group, -CF₃, and a dimethylamino group, and
R² is a hydrogen or a halogen.

In the disclosure, the term "substitution" refers to a reaction in which an atom or atomic group included in a molecule of a compound is replaced with another atom or atomic group.

In the disclosure, the term "chain" refers to a molecule having a chain structure, and the chain structure is a chemical structure in which carbon atoms are linked in a chain shape, and there are those having a straight chain shape and those having a branched shape.

In the disclosure, the term "cyclo or cyclic" refers to a structure in which linked ends in a skeleton of an organic compound are connected to form a ring shape.

In the disclosure, the term "chain or cyclic alkyl group" means a monovalent linear, branched, or cyclic saturated hydrocarbon residue having 1 to 20 carbon atoms and consisting of only carbon and hydrogen atoms. Examples of such an alkyl group include, but are not limited to, methyl, ethyl, propyl, isopropyl, butyl, isobutyl, 2-butyl, 3-butyl, pentyl, n-hexyl, a cyclobutyl group, a cyclopentyl group, a cyclohexyl group, and the like.

In the disclosure, the term "heterocycloalkyl group" generally refers to a saturated or unsaturated (but not aromatic) cyclohydrocarbon, which may be optionally unsubstituted, mono-substituted, or multi-substituted, and in a structure thereof, at least one is selected from a heteroatom of N, O, and S, and preferably may be any one of a piperidine, a piperazine, and a morpholine.

In the disclosure, the term "aryl group" means an unsaturated aromatic ring compound having 3 to 12 carbon atoms having a single ring (e.g., phenyl) or multiple condensed rings (e.g., naphthyl). Examples of such aryl groups include, but are not limited to, phenyl, naphthyl, and the like.

In the disclosure, the term "heteroaryl group" refers to a single ring or multiple condensed rings in which at least one of the atoms constituting the ring has a heteroatom of N, O or S. Examples of such heteroaryl groups include, but are not limited to, a pyridyl group, a pyrimidinyl group, a pyrazinyl group, an oxazolyl group, a furyl group, and a thiophenyl group.

In the disclosure, the "halogen group" may be fluorine (F), chloride (Cl), bromine (Br), or iodine (I).

In the disclosure, a "carbonyl group" refers to a -C(=O)- group, a "sulfonyl group" refers to a -S(=O)₂- group, a "sulfonamide group" refers to a -S(=O)₂-NH- group, an "acyl group" refers to a -C(=O)-R group, and an "alkylacyl group" refers to an acyl group in which R is an alkyl.

As a preferred embodiment of the disclosure, the compound represented by Formula 1 is preferably at least one selected from the group consisting of the following compounds:

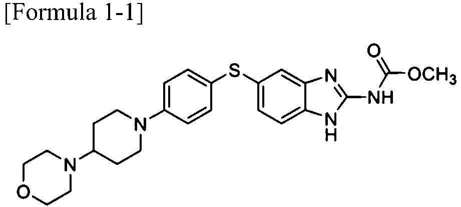

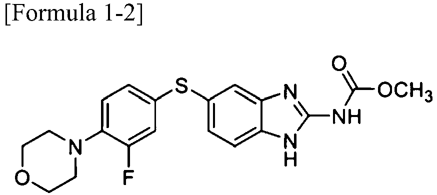

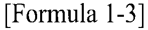

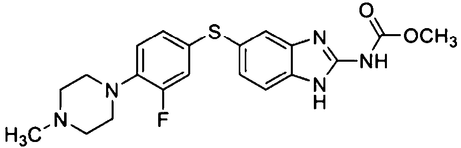

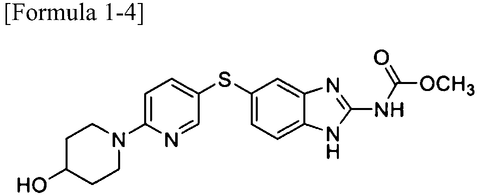

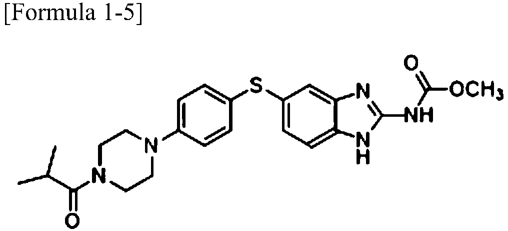

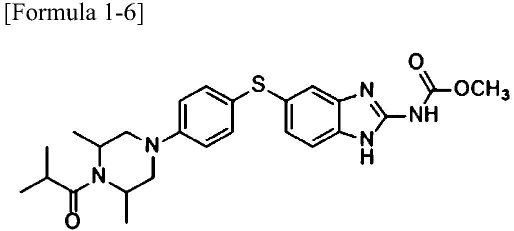

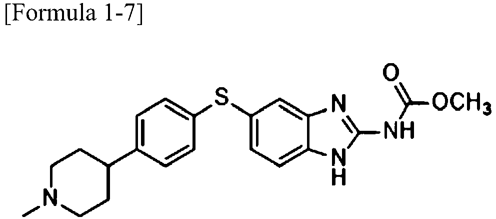

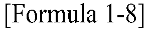

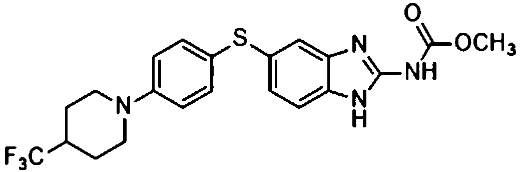

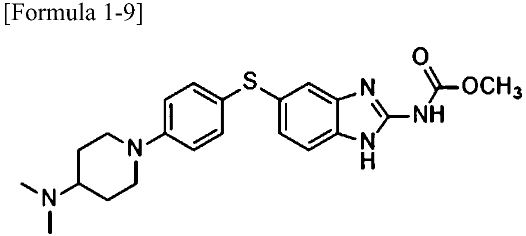

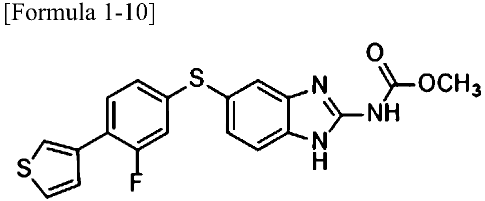

The thiobenzimidazole derivative, or the pharmaceutically acceptable salt thereof, of the disclosure is activated specifically in cancer cells to inhibit tubulin polymerization and induce apoptosis, and thus the thiobenzimidazole derivative, or the pharmaceutically acceptable salt thereof, may be used as a pharmaceutical composition for preventing or treating cancer including the thiobenzimidazole derivative, or the pharmaceutically acceptable salt thereof, as an active ingredient.

In the disclosure, the term "pharmaceutically acceptable salt" means a formulation of a compound that does not cause serious irritation to an organism to which the compound is administered and does not impair the biological activity and physical properties of the compound. The pharmaceutically acceptable salt may be obtained by reacting the compound of the disclosure with an inorganic acid such as hydrochloric acid, bromic acid, sulfuric acid, nitric acid, or phosphoric acid; a sulfonic acid such as methanesulfonic acid, ethanesulfonic acid, or p-toluenesulfonic acid; or an organic carboxylic acid such as tartaric acid, formic acid, citric acid, acetic acid, trichloroacetic acid, trifluoroacetic acid, capric acid, isobutanoic acid, malonic acid, succinic acid, phthalic acid, gluconic acid, benzoic acid, lactic acid, fumaric acid, maleic acid, or salicylic acid. In addition, the pharmaceutically acceptable salt may be obtained by reacting the compound of the disclosure with a base to form a salt, including salts such as ammonium salts; alkali metal salts such as sodium or potassium salts; alkaline earth metal salts such as calcium or magnesium salts; salts of organic bases such as dicyclohexylamine, N-methyl-D-glucamine, or tris(hydroxymethyl)methylamine; and amino acid salts such as arginine or lysine.

Furthermore, the thiobenzimidazole derivative or the pharmaceutically acceptable salt thereof may include not only pharmaceutically acceptable salts but also all salts, hydrates, and solvates capable of being prepared by conventional methods.

In addition, the disclosure may provide a method of preventing, treating, and/or diagnosing cancer, including administering the thiobenzimidazole derivative or a pharmaceutically acceptable salt thereof to a subject.

In the disclosure, the term "prevention" means any act of inhibiting or delaying the occurrence, spread or recurrence of the cancer by administering the composition of the disclosure, and "treatment" means any act of improving or beneficially changing the symptoms of the disease by administering the composition of the disclosure.

In the disclosure, the term "pharmaceutical composition" means a composition manufactured for the purpose of preventing or treating the above-described disease, and can be formulated and used in various forms according to conventional methods. For example, the pharmaceutical composition can be formulated into oral dosage forms such as powder, granules, tablets, capsules, suspensions, emulsions, and syrups, and can be formulated and used in the form of external preparations, suppositories, and sterile injectable solutions.

In the disclosure, "including as an active ingredient" means that the corresponding component is included in an amount necessary or sufficient to realize a desired biological effect. In actual application, the determination of the amount included as an active ingredient is an amount for treating a target disease, and may be determined by considering factors that do not cause other toxicity, and may vary according to various factors such as, for example, the disease or condition being treated, the form of the composition being administered, the size of the subject, or the severity of the disease or condition. A person skilled in the art to which the disclosure pertains can empirically determine the effective amount of an individual composition without undue experimentation.

In addition, the pharmaceutical composition of the disclosure may, depending on each formulation, additionally contain one or more pharmaceutically acceptable carriers in addition to the active ingredients described above.

The pharmaceutically acceptable carrier may be saline solution, sterile water, Ringer's solution, buffered saline, dextrose solution, maltodextrin solution, glycerol, ethanol, and mixtures of one or more of these components, and may further include other conventional additives such as antioxidants, buffers, and bacteriostatic agents, as needed. In addition, diluents, dispersants, surfactants, binders and lubricants may be additionally added to formulate the composition into injectable formulations such as aqueous solutions, suspensions and emulsions, pills, capsules, granules or tablets. Furthermore, the composition may be preferably formulated for each disease or ingredient using an appropriate method in the art or a method disclosed in Remington's Pharmaceutical Science (Mack Publishing Company, Easton PA).

The composition of the disclosure may be administered orally or parenterally in a pharmaceutically effective amount according to a desired method, and the term "pharmaceutically effective amount" of the disclosure means an amount that is sufficient to treat a disease with a reasonable benefit/risk ratio applicable to medical treatment and does not cause side effects, and the effective dose level may be determined according to factors including the patient's health condition, severity, activity of the drug, sensitivity to the drug, administration method, administration time, administration route and excretion rate, treatment period, drugs used in combination or concurrently, and other factors well known in the medical field.

Accordingly, the pharmaceutical composition of the disclosure may be administered to a subject to prevent, treat, and/or diagnose cancer, and the cancer may be skin cancer, breast cancer, uterine cancer, esophageal cancer, gastric cancer, brain tumor, colon cancer, rectal cancer, colorectal cancer, lung cancer, ovarian cancer, cervical cancer, endometrial cancer, vulvar cancer, renal cancer, hematologic cancer, pancreatic cancer, prostate cancer, testicular cancer, laryngeal cancer, head and neck cancer, thyroid cancer, liver cancer, bladder cancer, osteosarcoma, lymphoma, hematologic cancer, thymic cancer, urethral cancer, or bronchial cancer, but is not limited thereto, and preferably, may be a cancer having a characteristic in that acidity is higher than that of normal cells and cytotoxicity is inhibited by a tubulin polymerization inhibitor, and non-limiting examples thereof include breast cancer, preferably HER-2 positive breast cancer or triple-negative breast cancer.

In the disclosure, the term "subject" is not limited to a mammal such as livestock or a human that requires prevention, treatment, and/or diagnosis of the cancer, but may preferably be a human.

The pharmaceutical composition of the disclosure may be formulated in various forms for administration to a subject, and a representative form for parenteral administration is an injectable form, preferably an isotonic aqueous solution or suspension. Injectable formulations can be prepared according to techniques known in the art using suitable dispersing or wetting agents and suspending agents. For example, each component can be dissolved in saline or a buffer solution and formulated for injection. In addition, formulations for oral administration include, for example, ingestible tablets, buccal tablets, troches, capsules, elixirs, suspensions, syrups, and wafers, and these formulations may contain, in addition to active ingredients, diluents (e.g., lactose, dextrose, sucrose, mannitol, sorbitol, cellulose and/or glycine) and lubricants (e.g., silica, talc, stearic acid and its magnesium or calcium salts and/or polyethylene glycol). The tablets may contain binding agents such as magnesium aluminum silicate, starch paste, gelatin, tragacanth, methylcellulose, sodium carboxymethylcellulose and/or polyvinylpyrrolidine, and may optionally further contain disintegrants such as starch, agar, alginic acid or its sodium salt, absorbents, colorants, flavoring agents and/or sweetening agents. The formulations may be prepared by conventional mixing, granulation or coating methods.

In addition, the pharmaceutical composition of the disclosure may further include auxiliary agents such as preservatives, wetting agents, emulsifying agents, salts for osmotic pressure control or buffers, and other therapeutically useful substances, and may be formulated according to conventional methods.

The pharmaceutical composition according to the disclosure may be administered through various routes including oral, transdermal, subcutaneous, intravenous or intramuscular routes, and the dosage of the active ingredient may be appropriately selected according to various factors such as the route of administration, the patient's age, sex, weight, and the severity of the patient's condition. In addition, the composition of the disclosure may be administered in combination with known compounds that can enhance the desired effect.

As routes of administration for the pharmaceutical composition according to the disclosure, the pharmaceutical composition may be administered to humans and animals orally or parenterally such as intravenously, subcutaneously, intranasally, or intraperitoneally. Oral administration also includes sublingual application. Parenteral administration includes injection methods such as subcutaneous injection, intramuscular injection, and intravenous injection, and instillation methods.

In the pharmaceutical composition of the disclosure, the total effective amount of the thiobenzimidazole derivative or a pharmaceutically acceptable salt thereof according to the disclosure may be administered to a patient as a single dose, or may be administered by a fractionated treatment protocol in which multiple doses are administered over a long period of time. The pharmaceutical composition of the disclosure may vary the content of the active ingredient depending on the severity of the disease, but may typically be administered several times a day at an effective dose of 100 µg to 3,000 mg per administration for adults. However, regarding the concentration of the thiobenzimidazole derivative or the pharmaceutically acceptable salt thereof, an effective dosage for a patient may be determined by considering various factors such as age, body weight, health condition, sex, severity of the disease, diet, and excretion rate of a patient, as well as a route of administration and a frequency of treatment of the drug.

In addition, the pharmaceutical composition according to the disclosure is not particularly limited in its formulation, administration route, and administration method as long as it exhibits the effects of the disclosure, and the pharmaceutical composition of the disclosure may additionally include a known anticancer agent in addition to the thiobenzimidazole derivative or a pharmaceutically acceptable salt thereof as an active ingredient, and may be used in combination with other known treatments for the treatment of these diseases.

The terminology used herein is for the purpose of describing particular embodiments only and is not to be limiting of the embodiments. The singular forms "a", "an", and "the" are intended to include the plural forms as well, unless the context clearly indicates otherwise. It will be further understood that the terms "comprises/comprising" and/or "includes/including" when used herein, specify the presence of stated features, integers, steps, operations, elements, and/or components, but do not preclude the presence or addition of one or more other features, integers, steps, operations, elements, components and/or groups thereof.

Unless otherwise defined, all terms including technical and scientific terms used herein have the same meaning as commonly understood by one of ordinary skill in the art to which the examples belong. Terms, such as those defined in commonly used dictionaries, are to be interpreted as having a meaning that is consistent with their meaning in the context of the relevant art and the present disclosure, and are not to be interpreted in an idealized or overly formal sense unless expressly so defined herein.

The disclosure can be modified in various ways and has various embodiments. Specific embodiments are illustrated in the drawings and described in detail in the detailed description below. However, this is not intended to limit the disclosure to specific embodiments, and it should be understood to include all modifications, equivalents, or substitutes that fall within the idea and scope of the disclosure. In the description of embodiments, detailed description of well-known related technologies will be omitted when it is deemed that such description will cause ambiguous interpretation of the disclosure.

### MODE FOR CARRYING OUT THE DISCLOSURE

### Preparation Example. Synthesis of Methyl (5-(phenylthio)-1H-benzo[d]imidazol-2-yl)carbamate Derivatives

The compounds of the disclosure were prepared by a reaction represented by the following reaction scheme:

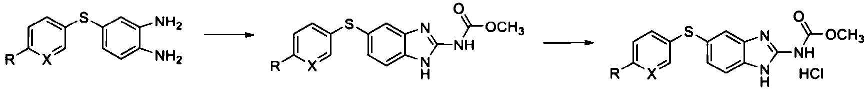

### 1-1. Synthesis of Methyl (5-((4-(4-morpholinopiperidin-1-yl)phenyl)thio)-1H-benzo[d]imidazol-2-yl)carbamate 5 (Formula 1-1)

1,3-Bis(methoxycarbonyl)-S-methylisothiourea (2.5 eq) was added to a solution of 4-((4-(4-morpholinopiperidin-1-yl)phenyl)thio)benzene-1,2-diamine (0.241 g, 1.07 mmol) in 5% acetic acid in EtOH (5 mL), and the mixture was heated and stirred at 90°C for 20 hours, and then the solvent was removed by evaporation under reduced pressure.

The reaction product was sufficiently cooled and left standing at room temperature to produce a solid, which was filtered and simultaneously washed with MeOH. At this time, the solid was washed with MeOH until the sulfur odor of the remaining urea was completely removed and dried to obtain methyl (5-((4-(4-morpholinopiperidin-1-yl)phenyl)thio)-1H-benzo[d]imidazol-2-yl)carbamate (0.250 g) as a white solid.
Yield 85%
¹H NMR (400 MHz, DMSO-d₆) δ 11.72(m, 2H), 7.34(d, J=8.0Hz, 1H), 7.289s, 1H), 7.21(d, J=8.8Hz, 2H), 7.02(m, 1H), 6.93(d, J=8.8Hz, 2H), 3.74(s, 3H), 3.72(m, 2H), 3.56(m, 4H), 2.70(t, 2H), 2.47(m, 4H), 2.26(m, 1H), 1.85(d, J=10.8Hz, 2H), 1.45(m, 2H)

### 1-2. Synthesis of Methyl (5-((3-fluoro-4-morpholinophenyl)thio)-1H-benzo[d]imidazol-2-yl)carbamate (Formula 1-2)

1,3-Bis(methoxycarbonyl)-S-methylisothiourea (2.5 eq) was added to a solution of 4-((3-fluoro-4-morpholinophenyl)thio)benzene-1,2-diamine (0.230 g, 0.72 mmol) in 5% acetic acid in EtOH (5 mL), and the mixture was heated and stirred at 90°C for 5 hours, and then the solvent was removed by evaporation under reduced pressure.

The reaction product was sufficiently cooled and left standing at room temperature to produce a solid, which was filtered and simultaneously washed with MeOH. At this time, the solid was washed with MeOH until the sulfur odor of the remaining urea was completely removed and dried to obtain methyl (5-((3-fluoro-4-morpholinophenyl)thio)-1H-benzo[d]imidazol-2-yl)carbamate (0.230 g) as a white solid.
Yield 79%
¹H NMR (400 MHz, DMSO-d₆) δ 11.82(m, 1H), 11.64(m, 1H), 7.46(s, 1H), 7.43(d, J=8.0Hz, 1H), 7.16(d, J=8.4Hz, 1H), 6.97(m, 3H), 3.75(s, 3H), 3.71(m, 4H), 2.97(m, 4H),

### 1-3. Synthesis of Methyl (5-((3-fluoro-4-(4-methylpiperazin-1-yl)phenyl)thio)-1H-benzo[d]imidazol-2-yl)carbamate (Formula 1-3)

1,3-Bis(methoxycarbonyl)-S-methylisothiourea (0.234 g, 1.13 mmol) was added to a solution of 4-((3-fluoro-4-(4-methylpiperazin-1-yl)phenyl)thio)benzene-1,2-diamine (0.150 g, 0.45 mmol) in 5% acetic acid in EtOH (3 mL), and the mixture was heated and stirred at 90°C for 5 hours, and then the solvent was removed by evaporation under reduced pressure.

The reaction product was sufficiently cooled and left standing at room temperature to produce a solid, which was filtered and simultaneously washed with MeOH. At this time, the solid was washed with MeOH until the sulfur odor of the remaining urea was completely removed, and then separated by column chromatography using silica gel with a developing solvent of methylene chloride and methanol (9:1, v/v/), and dried to obtain methyl (5-((3-fluoro-4-(4-methylpiperazin-1-yl)phenyl)thio)-1H-benzo[d]imidazol-2-yl)carbamate (0.110 g) as a white solid.
Yield 59%
¹H NMR (400 MHz, DMSO-d₆) δ 11.95(m, 1H), 11.40(m, 1H), 7.45(m, 2H), 7.15(d, J=8.4Hz, 1H), 6.96(s, 2H), 6.93(m, 1H), 3.75(s, 3H), 3.65(m, 4H), 2.97(m, 4H), 2.20(s, 3H)

### 1-4. Synthesis of Methyl (5-((6-(4-hydroxypiperidin-1-yl)pyridin-3-yl)thio)-1H-benzo[d]imidazol-2-yl)carbamate (Formula 1-4)

1-(5-((3,4-diaminophenyl)thio)pyridin-2-yl)piperidin-4-ol and 1,3-Bis(methoxycarbonyl)-S-methylisothiourea were reacted in the same manner as described above to obtain methyl (5-((6-(4-hydroxypiperidin-1-yl)pyridin-3-yl)thio)-1H-benzo[d]imidazol-2-yl)carbamate.
Yield 84%
¹H NMR (400 MHz, DMSO-d₆) δ 11.82(m, 1H), 11.33(m, 1H), 8.18(s, 1H), 7.55(d, J=8.8Hz, 1H), 7.33(d, J=8.4Hz, 1H), 7.24(s, 1H), 7.01(d, J=8.4Hz, 1H), 6.87(d, J=8.8Hz, 1H), 4.72(s, 1H), 4.02(m, 2H), 3.73(s, 3H), 3.70(m, 1H), 3.12(m, 2H), 1.78(m, 2H), 1.35(m, 2H)

### 1-5. Synthesis of Methyl (5-((4-(4-isobutyrylpiperazin-1-yl)phenyl)thio)-1H-benzo[d]imidazol-2-yl)carbamate (Formula 1-5)

1-(4-(4-((3,4-diaminophenyl)thio)phenyl)piperazin-1-yl)-2-methylpropan-1-one and 1,3-Bis(methoxycarbonyl)-S-methylisothiourea were reacted in the same manner as described above to obtain methyl (5-((4-(4-isobutyrylpiperazin-1-yl)phenyl)thio)-1H-benzo[d]imidazol-2-yl)carbamate.
Yield 74%
¹H NMR (400 MHz, DMSO-d₆) δ 11.73(m, 2H), 7.35(m, 2H), 7.23(d, J=8.8Hz, 2H), 7.04(m, 1H), 6.96(d, J=8.8Hz, 2H), 3.74(s, 3H), 3.62(d, J=18.0Hz, 4H), 3.16(d, J=21.6Hz, 4H), 2.90(q, 1H), 1.01(d, J=6.8Hz, 6H)

### 1-6. Synthesis of Methyl (5-((4-(4-isobutyryl-3,5-dimethylpiperazin-1-yl)phenyl)thio)-1H-benzo[d]imidazol-2-yl)carbamate (Formula 1-6)

1-(4-(4-((3,4-diaminophenyl)thio)phenyl)-2,6-dimethylpiperazin-1-yl)-2-methylpropan-1-one and 1,3-Bis(methoxycarbonyl)-S-methylisothiourea were reacted in the same manner as described above to obtain methyl (5-((4-(4-isobutyryl-3,5-dimethylpiperazin-1-yl)phenyl)thio)-1H-benzo[d]imidazol-2-yl)carbamate.
Yield 87%
¹H NMR (400 MHz, DMSO-d₆) δ 11.72(m, 2H), 7.33(d, J=9.6Hz, 2H), 7.25(d, J=8.8Hz, 2H), 7.04(d, J=8.0Hz, 1H), 6.97(d, J=8.8Hz, 2H), 4.52(bs, 1H), 4.19(bs, 1H), 3.74(s, 3H), 3.54(m, 2H), 2.85(m, 3H), 1.32(bs, 3H), 1.19(bs, 3H), 1.04((d, J=6.8Hz, 6H)

### 1-7. Synthesis of Methyl (5-((4-(1-methylpiperidin-4-yl)phenyl)thio)-1H-benzo[d]imidazol-2-yl)carbamate (Formula 1-7)

4-((4-(1-methylpiperidin-4-yl)phenyl)thio)benzene-1,2-diamine and 1,3-Bis(methoxycarbonyl)-S-methylisothiourea were reacted in the same manner as described above to obtain methyl (5-((4-(1-methylpiperidin-4-yl)phenyl)thio)-1H-benzo[d]imidazol-2-yl)carbamate.
Yield 61%
¹H NMR (400 MHz, DMSO-d₆) δ 11.82(m, 2H), 7.48(s, 1H), 7.43(d, J=8.0Hz, 1H), 7.18(d, J=8.4Hz, 3H), 7.09(d, J=8.0Hz, 2H), 3.75(s, 3H), 2.84(d, J=11.2Hz, 2H), 2.35(m, 1H), 2.16(s, 3H), 1.91(m, 2H), 1.68(m, 2H), 1.60(m, 2H)

### 1-8. Synthesis of Methyl (5-((4-(4-(trifluoromethyl)piperidin-1-yl)phenyl)thio)-1H-benzo[d]imidazol-2-yl)carbamate (Formula 1-8)

4-((4-(4-(trifluoromethyl)piperidin-1-yl)phenyl)thio)benzene-1,2-diamine and 1,3-Bis(methoxycarbonyl)-S-methylisothiourea were reacted in the same manner as described above to obtain methyl (5-((4-(4-(trifluoromethyl)piperidin-1-yl)phenyl)thio)-1H-benzo[d]imidazol-2-yl)carbamate.
Yield 68%
¹H NMR (400 MHz, DMSO-d₆) δ 11.90(m, 1H), 11.33(m, 1H), 7.35(m, 1H), 7.30(d, J=9.2Hz, 2H), 7.04(d, J=8.0Hz, 1H), 6.96(d, J=8.0Hz, 2H), 3.81(d, J=12.8Hz, 2H), 3.74(s, 3H), 2.72(t, 2H), 2.70(m, 1H), 1.88(d, J=12.4Hz, 2H), 1.53(m, 2H)

### 1-9. Synthesis of Methyl (5-((4-(4-(dimethylamino)piperidin-1-yl)phenyl)thio)-1H-benzo[d]imidazol-2-yl)carbamate (Formula 1-9)

4-((4-(4-(dimethylamino)piperidin-1-yl)phenyl)thio)benzene-1,2-diamine and 1,3-Bis(methoxycarbonyl)-S-methylisothiourea were reacted in the same manner as described above to obtain methyl (5-((4-(4-(dimethylamino)piperidin-1-yl)phenyl)thio)-1H-benzo[d]imidazol-2-yl)carbamate.
Yield 73%
¹H NMR (400 MHz, DMSO-d₆) δ 11.63(m, 2H), 7.34(d, J=8.0Hz, 1H), 7.28(s, 1H), 7.21(d, J=8.8Hz, 2H), 7.02(d, J=6.8Hz, 1H), 6.93(d, J=9.2Hz, 2H), 3.73(s, 3H), 3.70(m, 2H), 2.70(t, 2H), 2.17(s, 6H), 2.16(m, 1H), 1.82(d, J=12.4Hz, 2H), 1.43(q, 2H)

### 1-10. Synthesis of Methyl (5-((3-fluoro-4-(thiophen-3-yl)phenyl)thio)-1H-benzo[d]imidazol-2-yl)carbamate (Formula 1-10)

4-((3-fluoro-4-(thiophen-3-yl)phenyl)thio)benzene-1,2-diamine and 1,3-Bis(methoxycarbonyl)-S-methylisothiourea were reacted in the same manner as described above to obtain methyl (5-((3-fluoro-4-(thiophen-3-yl)phenyl)thio)-1H-benzo[d]imidazol-2-yl)carbamate.
Yield 83%
¹H NMR (400 MHz, DMSO-d₆) δ 11.99(bs, 1H), 11.55(bs, 1H), 7.79(s, 1H), 7.78(m, 3H), 7.65(d, J=8.4Hz, 1H), 7.52(d, J=8.0Hz, 1H), 7.44(d, J=4.8Hz, 1H), 7.28(d, J=8.0Hz, 1H), 6.95(s, 2H), 3.77(s, 3H)

### 1-11. Synthesis of Methyl (5-((6-(4-hydroxypiperidin-1-yl)pyridin-3-yl)thio)-1H-benzo[d]imidazol-2-yl)carbamate hydrochloride salt (hydrochloride of Formula 1-4)

Hydrogen chloride gas was injected at 2-minute intervals into a suspension of methyl (5-((6-(4-hydroxypiperidin-1-yl)pyridin-3-yl)thio)-1H-benzo[d]imidazol-2-yl)carbamate (100 mg, 0.25 mmol) in methanol (35 mL) until the pH reached 1 or below. The reaction product was evaporated under reduced pressure to obtain a product in an oil form, and then isopropyl alcohol (IPA, 20 mL) was added thereto, and the product was dissolved by heating and then cooled to room temperature, whereby a solid suspension was produced. Isopropyl ether (20 mL) was added thereto in portions while stirring for 2 hours, and then the mixture was filtered. At this time, a washing was performed with a mixed solvent of IPA and isopropyl ether (IPE), and a hot air drying was performed at 50°C for 1 hour, and continuously, a vacuum drying was performed at 40°C for 2 hours to obtain a white solid (90 mg).
Yield 82%
1H NMR (D2O, 400 MHz) δ 7.81-6.80(m, 6H), 3.71(s, 3H), 3.90(m, 2H), 3.69(m, 1H), 3.20(m, 2H), 1.84(m, 2H), 1.37(m, 2H).

### Example 1. Confirmation of Cell Viability of Thiobenzimidazole Derivatives Against Breast Cancer Cell Lines

MDA-MB-231 (Cell seeding numbers: 0.8(M231) x 10⁴ cells/wells (confluency ≧ 25%), which is a human triple-negative breast cancer (TNBC) cell line, and JIMT-1 (Cell seeding numbers: 0.8(JIMT) x 10⁴ cells/wells (confluency ≧ 25%)), which is a HER2 positive breast cancer (HER2+ BC) cell line, were used in the experiment.

The above cell lines were cultured in an environment of 5% CO₂ and 37°C in a Dulbecco's modified Eagle's medium (DMEM) including a 10% fetal bovine serum (FBS), a streptomycin-penicillin (100 U/mL), and a Fungizone (0.625 µg/mL), respectively.

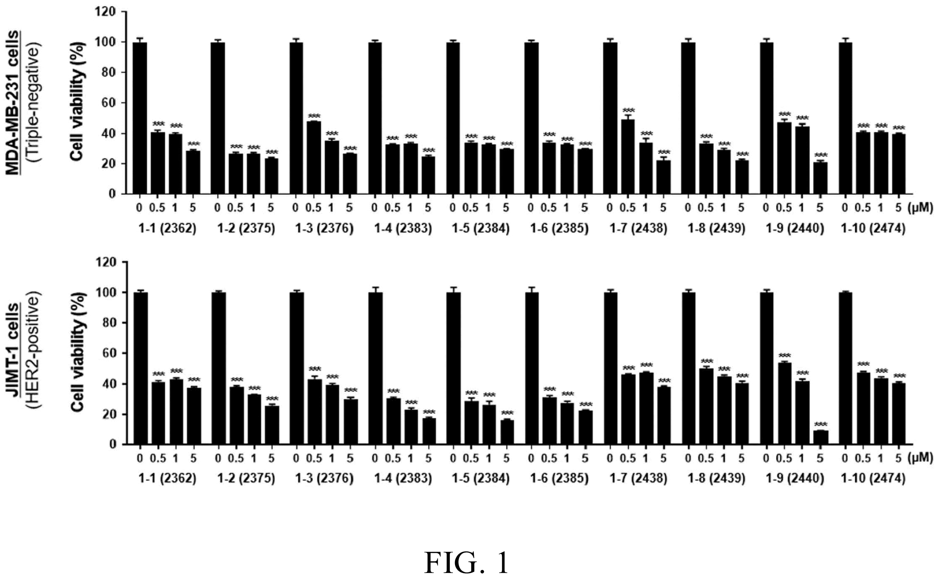

The human breast cancer cell lines MDA-MB-231 and JIMT-1 were treated with thiobenzimidazole derivatives of Formulas 1-1 to 1-10 at concentrations of 0, 0.5, 1, and 5 µM, respectively, for 72 hours, and then cell viability was measured using the MTS assay technique. In the MTS assay, cells were attached to a 96-well plate for 24 hours, treated with the thiobenzimidazole derivative for 72 hours, developed with MTS (3-(4,5-dimethylthiazol-2-yl)-5-(3-carboxymethoxyphenyl)-2-(4-sulfophenyl)-2H-tetrazolium) for 4 hours, and then absorbance was measured at 490 nm using a Spectramax Plus384 microplate analyzer. The results are illustrated in FIG. 1, and as illustrated in the drawing, it was confirmed that the thiobenzimidazole derivatives of Formula 1-1 to 1-10 inhibited cell viability in a concentration-dependent manner in the human breast cancer cell lines MDA-MB-231 and JIMT-1.

### Example 2. Confirmation of Cell Viability and IC₅₀ Values of Thiobenzimidazole Derivatives Against Breast Cancer Cell Lines

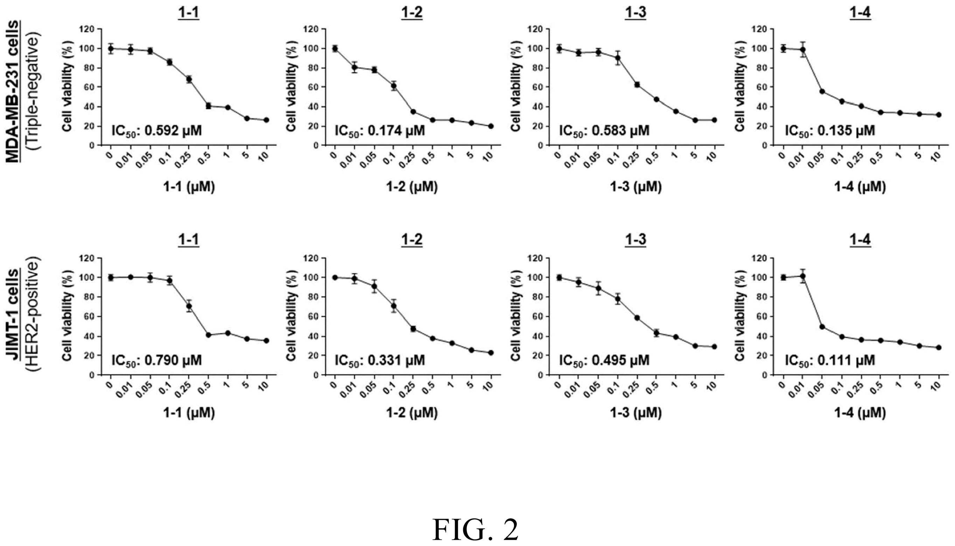

The MDA-MB-231 and JIMT-1 cell lines were treated with thiobenzimidazole derivatives (Formula 1-1 to 1-4) of the disclosure at concentrations of 0, 0.01, 0.05, 0.1, 0.25, 0.5, 1, 5, and 10 µM, respectively, and then cell viability and IC₅₀ values therefrom were confirmed. The preparation method of the cell lines and the experimental method were the same as those performed in Example 1. The results are illustrated in FIG. 2, and it was confirmed that the thiobenzimidazole derivatives of Formula 1-1 to Formula 1-4 had low IC₅₀ values of 0.592 µM, 0.174 µM, 0.583 µM, and 0.135 µM, respectively, in the MDA-MB-231 cell line.

Although the embodiments are described with reference to the limited drawings, one of ordinary skill in the art may apply various technical modifications and variations based thereon. For example, suitable results may be achieved if the described techniques are performed in a different order, and/or if components in a described system, structure, device, or circuit are combined in a different manner, and/or replaced or supplemented by other components or their equivalents.

Therefore, other implementations, other preparation examples, and equivalents to the claims are also within the scope of the following claims.

## Claims

1. A thiobenzimidazole derivative represented by [Formula 1], or a pharmaceutically acceptable salt thereof: 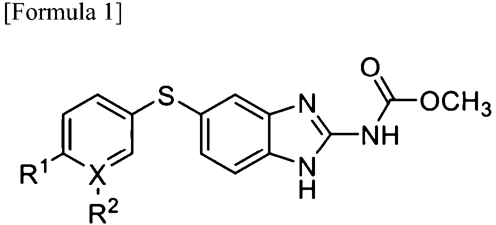 wherein, in Formula 1,
X is any one selected from C and N,
R¹ is any one selected from a substituted or unsubstituted C₃ to C₂₀ cycloalkyl, a substituted or unsubstituted C₃ to C₂₀ heterocycloalkyl, a substituted or unsubstituted C₃ to C₂₀ aryl, and a substituted or unsubstituted C₃ to C₂₀ heteroaryl,
when the cycloalkyl, the heterocycloalkyl, the aryl, or the heteroaryl is substituted, the cycloalkyl, the heterocycloalkyl, the aryl, or the heteroaryl is substituted with one or more selected from a heterocycloalkyl group, a C₁ to C₆ alkyl group, a hydroxy group, an alkylacyl group, -CF₃, and a dimethylamino group, and
R² is a hydrogen or a halogen.

2. The thiobenzimidazole derivative or the pharmaceutically acceptable salt thereof of claim 1,
wherein, in [Formula 1],
R¹ is any one selected from the group consisting of a substituted or unsubstituted piperidine, a substituted or unsubstituted morpholine, a substituted or unsubstituted piperazine, and a substituted or unsubstituted thiophene, and
when the piperidine, the morpholine, the piperazine, or the thiophene is substituted, the piperidine, the morpholine, the piperazine, or the thiophene is substituted with one or more selected from a morpholine group, a methyl group, a hydroxy group, an isobutylacyl group, -CF₃, and a dimethylamino group.

3. The thiobenzimidazole derivative or the pharmaceutically acceptable salt
thereof of claim 1, wherein the thiobenzimidazole derivative represented by [Formula 1] is any one selected from the group consisting of the following compounds:

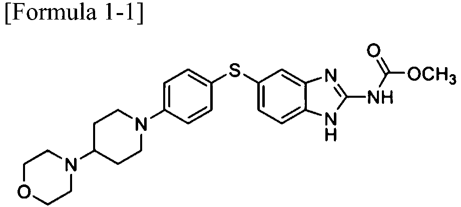

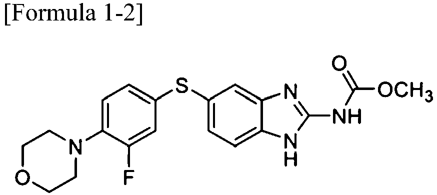

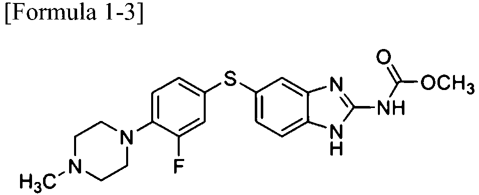

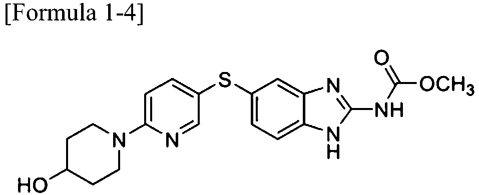

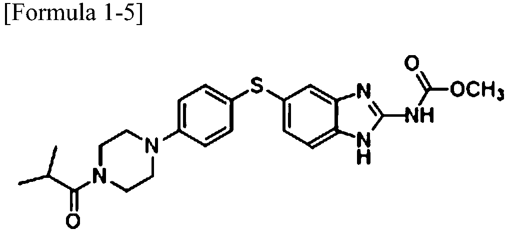

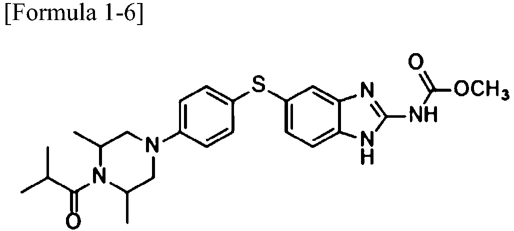

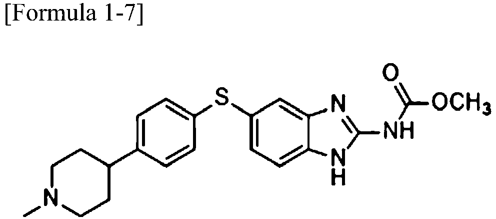

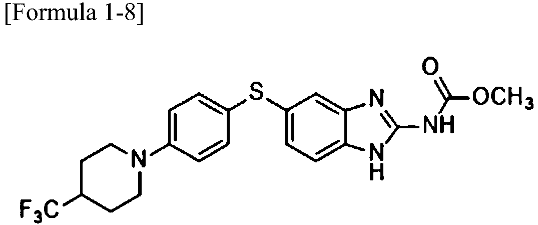

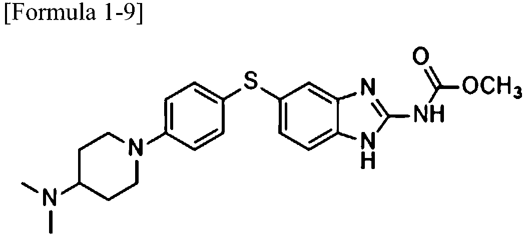

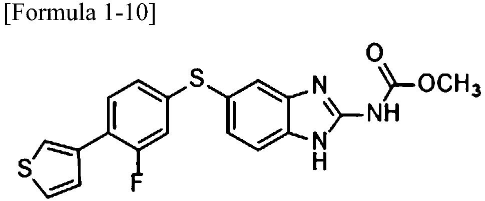

4. The thiobenzimidazole derivative or the pharmaceutically acceptable salt thereof of claim 1, wherein the thiobenzimidazole derivative represented by [Formula 1] is any one selected from the group consisting of the following compounds:

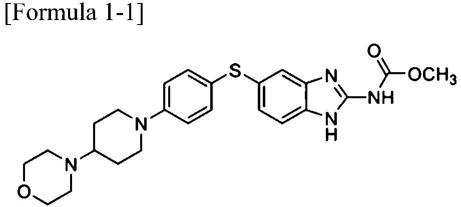

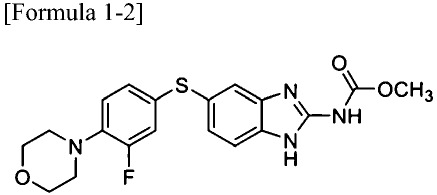

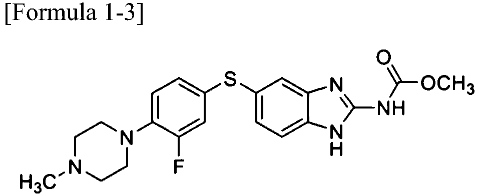

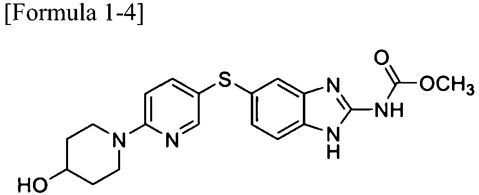

5. The thiobenzimidazole derivative or the pharmaceutically acceptable salt thereof of claim 1, wherein the thiobenzimidazole derivative inhibits tubulin polymerization.

6. The thiobenzimidazole derivative or the pharmaceutically acceptable salt thereof of claim 1, wherein the pharmaceutically acceptable salt of the thiobenzimidazole derivative is one or more selected from the group consisting of a hydrochloride, a bromate, a sulfate, a phosphate, a nitrate, a citrate, an acetate, a lactate, a tartrate, a maleate, a gluconate, a succinate, a formate, a trifluoroacetate, an oxalate, a fumarate, a glutarate, an adipate, a methanesulfonate, a benzenesulfonate, a p-toluenesulfonate, a camphorsulfonate, a sodium salt, a potassium salt, a lithium salt, a calcium salt, and a magnesium salt.

7. The thiobenzimidazole derivative or the pharmaceutically acceptable salt thereof of claim 1, wherein the pharmaceutically acceptable salt of the thiobenzimidazole derivative is a hydrochloride.

8. A pharmaceutical composition for preventing or treating cancer, the pharmaceutical composition comprising, as an active ingredient, the thiobenzimidazole derivative of any one of claims 1 to 7, or a pharmaceutically acceptable salt thereof.

9. The pharmaceutical composition of claim 8, wherein the pharmaceutical composition induces apoptosis by causing cell cycle arrest in cancer cells.

10. The pharmaceutical composition of claim 8, wherein the cancer is one or more selected from the group consisting of skin cancer, breast cancer, uterine cancer, esophageal cancer, gastric cancer, brain tumor, colon cancer, rectal cancer, colorectal cancer, lung cancer, ovarian cancer, cervical cancer, endometrial cancer, vulvar cancer, renal cancer, hematological cancer, pancreatic cancer, prostate cancer, testicular cancer, laryngeal cancer, head and neck cancer, thyroid cancer, hepatic cancer, bladder cancer, osteosarcoma, lymphoma, hematological cancer, thymic cancer, urethral cancer, and bronchial cancer.

11. The pharmaceutical composition of claim 8, wherein the cancer is HER-2 positive breast cancer or triple-negative breast cancer.
